# FASCICULE DE BREVET EUROPEEN

(11) **EP 2 655 281 B1**
(45) Date de publication et mention de la délivrance du brevet: **04.10.2017**
(21) Numéro de dépôt: 11815476.4
(22) Date de dépôt: 20.12.2011
(51) Int. Cl.: C04B 24/02, C04B 24/26, C04B 40/00, C04B 28/02, C04B 103/50, C04B 103/32

(54) **COMPOSITION CIMENTAIRE RENFERMANT UN ADDITIF ANTI-MOUSSE HYDROSOLUBLE, SOLUTION AQUEUSE CONTENANT LEDIT ADDITIF ET LEUR UTILISATION DANS DES MORTIERS OU BETONS**
WASSERLÖSLICHEN SCHAUMHEMMUNGSZUSATZ ENTHALTENDE ZEMENTZUSAMMENSETZUNG, DIESEN ENTHALTENDE WÄSSRIGE LÖSUNG UND VERWENDUNG FÜR BETON ODER MÖRTEL
CEMENT COMPOSITION COMPRISING A WATER-SOLUBLE ANTIFOAM ADDITIVE, AQUEOUS SOLUTION CONTAINING SAID ADDITIVE USE THEREOF IN MORTARS OR CONCRETES

(30) Priorité: 23.12.2010 FR 1061184
(43) Date de publication de la demande: 30.10.2013
(73) Titulaire: Ciments Français, 92800 Puteaux (FR)
(72) Inventeur: MEHALEBI, Soraya, F-78300 Poissy (FR); DESMOTZ, Anne-Elisabeth, F-78955 Carrieres sous Poissy (FR)
(74) Mandataire: Le Cloirec, Claudine
(86) Numéro de dépôt international: PCT/FR2011/053099
(87) Numéro de publication internationale: WO 2012/085452

(56) Documents cités:
- EP-A1- 0 752 465
- EP-A2- 0 519 747
- WO-A1-2005/121252
- WO-A2-02/090285
- FR-A1- 2 760 004
- FR-A1- 2 866 330
- US-A1- 2005 257 720
- US-A1- 2008 280 786
- DATABASE WPI Week 198209, Septembre 1982 Derwent Publications Ltd., London, GB; AN 1982-17654E, XP002765809 -& SU 829 874 A1 (AS UKR COLLOID CHEM) 18 Mai 1981

## Description

La présente invention concerne une composition cimentaire renfermant un additif anti-mousse, une solution aqueuse incorporant ledit additif, ainsi que leur utilisation dans des mortiers ou bétons.

La formulation des bétons à hautes performances fait souvent appel aux plastifiants et aux superplastifiants soit pour augmenter la fluidité de la composition cimentaire de base à apport en eau constant permettant l'amélioration de sa maniabilité, soit pour diminuer la teneur en eau de la composition cimentaire à consistance égale en vue d'améliorer les résistances mécaniques et la durabilité.

Parmi les plastifiants et superplastifiants, on utilise très souvent des molécules choisies parmi les polynaphtalènes sulfonates, les polymélamines sulfonates, les lignosulfonates (LS) et les polycarboxylates, notamment les polycarboxylates avec des chaînes latérales polyoxyde d'éthylène (PC-POE) introduits généralement sous forme d'une solution aqueuse dans la composition cimentaire lors du gâchage et malaxage de bétons et de mortiers. Les molécules de plastifiants et superplastifiants présentent des propriétés de surface, ce qui provoque un entraînement de l'air dans la composition cimentaire. Cet air occlus induit une modification de la rhéologie de la composition cimentaire et une chute de résistance mécanique non négligeable tant à court terme qu'à long terme du mortier ou du béton ainsi produit.

Ce phénomène est observé de manière plus importante avec les molécules des familles LS et PC-POE.

Pour limiter l'inclusion d'air dans la composition cimentaire, des anti-mousses sont généralement introduits directement dans les solutions de superplastifiants de type LS ou PC-POE de manière à prévenir la formation des bulles d'air ou à favoriser leur destruction si celles-ci se forment.

Deux contraintes sont liées à ces molécules anti-moussantes. La première concerne la nécessité de disperser l'anti-mousse dans la solution aqueuse de superplastifiant pour commercialiser un produit unique assurant les deux fonctions (fluidification de la composition cimentaire sans entraînement d'air trop important), tandis que la seconde contrainte touche à l'efficacité même de la molécule anti-mousse vis-à-vis de la prévention ou de la destruction de l'air occlus.

Les agents anti-mousses ou les agents démoussants sont des molécules identiques, qui ne diffèrent que par leur mode d'introduction dans le milieu. En effet, les agents anti-mousses sont introduits avant l'apparition de la mousse pour prévenir la formation d'interfaces gaz/liquide stables, tandis que les agents démoussants sont introduits après formation de la mousse pour l'éliminer.

La présente invention concerne plutôt la formulation des agents anti-mousse, c'est-à-dire des additifs destinés à être introduits en même temps que la solution de superplastifiant, dans la composition cimentaire, à base de ciment et d'eau, au moment de son malaxage.

Les agents anti-mousses sont en général des formulations hydrophobes se présentant sous forme de gouttelettes de petites tailles insolubles au-delà d'une concentration limite dans le liquide constituant la phase continue, ici la phase aqueuse. L'agent anti-mousse est donc par nature insoluble en milieu aqueux dans lequel il forme une émulsion.

Pour être efficace l'agent anti-mousse doit ensuite migrer vers l'interface air/film liquide. Lors de cette migration, les interfaces anti-mousses/film liquide et air/film liquide sont remplacées par une interface anti-mousse/air. Ce passage requiert un agent anti-mousse hydrophobe puisqu'une formulation hydrophile resterait au cour de la phase aqueuse.

L'utilisation des agents anti-mousses pour les compositions cimentaires telles que les coulis de ciment, mortiers ou bétons nécessitent donc de disperser l'agent anti-mousse dans la solution aqueuse de superplastifiants. Puisque l'anti-mousse est par nature une molécule hydrophobe, insoluble dans le milieu aqueux, il est nécessaire de réaliser une émulsion d'anti-mousse dans la solution aqueuse de superplastifiants.

Pour être commercialisable, cette émulsion d'anti-mousse dans le superplastifiant doit être stable sur toute la durée de stockage (soit un an environ) et sur la plage de températures auxquelles sont usuellement exposés les superplastifiants (5-40 °C sachant qu'en général l'émulsion est d'autant moins stable que la température est plus élevée).

Par ailleurs, l'anti-mousse doit également être efficace dans le milieu cimentaire sur toute la durée de mise en oeuvre (soit de 15 minutes à plusieurs heures).

En outre, une perte d'activité peut être engendrée par une dégradation de l'anti-mousse dans le milieu extrêmement basique des compositions cimentaires. Les molécules d'anti-mousse doivent donc être capables d'y résister.

D'autre part, "l'épuisement" de l'anti-mousse au cours du temps (se traduisant par une perte d'efficacité dans le temps) est un phénomène bien connu qui ne doit pas intervenir sur la période de mise en oeuvre des compositions cimentaires. Ce phénomène résulte souvent de la réduction de taille des gouttelettes anti-mousse au cours du temps du fait des cisaillements auxquels elles sont soumises.

En outre, l'anti-mousse doit pouvoir être utilisé avec le matériel classique de dosage. Certains agents anti-mousses, tel que le tributylphosphate, provoquent, par exemple, une dégradation de certains corps de pompe. Le document US 2005/025772 décrit la combinaison d'un agent anti-mousse avec un agent de solubilisation aminé.

En conséquence, un premier but de l'invention est de proposer un agent anti-mousse stable en solution aqueuse de superplastifiant, sur l'ensemble de la durée de stockage avant la commercialisation, c'est-à-dire ne présentant pas de séparation de phases, pendant plusieurs mois.

Un autre but de l'invention est de proposer un anti-mousse efficace pour empêcher ou limiter l'incorporation d'air occlus dans des compositions cimentaires tels que coulis, mortiers ou bétons, lors du gâchage et/ou du malaxage.

A cet effet, la présente invention concerne une composition cimentaire, à base de ciment et d'eau, préparée avec adjonction de superplastifiant, caractérisée en ce qu'elle renferme un additif anti-mousse se présentant sous la forme d'un ester d'alcool gras soluble en milieu aqueux à pH acide et hydrolysable lors de son incorporation dans ladite composition cimentaire, apte à libérer ledit alcool gras, l'alcool gras étant une molécule active anti-mousse.

Cet ester d'alcool gras étant hydrolysable lors de son incorporation dans ladite composition cimentaire, c'est-à-dire apte à libérer au moins une molécule active anti-mousse, il constitue un précurseur de ladite molécule active anti-mousse.

Le fait que l'additif anti-mousse soit soluble en milieu aqueux à pH acide, de préférence inférieur à 6, permet de résoudre le problème de la stabilité de cet additif dans les solutions aqueuses de superplastifiants, sans séparation de phase. On a ainsi également une homogénéisation aisée et donc une bonne répartition de l'anti-mousse dans l'ensemble de la solution de superplastifiant.

Les compositions cimentaires, telles que coulis de ciment, mortier ou béton frais, sont des milieux très basiques dont le pH de la solution interstitielle lors du gâchage est très souvent supérieur à 12. A cette valeur de pH, l'hydrolyse du précurseur anti-mousse in situ permet donc de libérer la molécule active au sein même de la composition cimentaire, là où sa présence est requise pour limiter ou empêcher la formation de bulles d'air notamment lors du gâchage avec l'eau et le ciment et du malaxage.

De préférence, l'ester d'alcool gras est un ester d'alcool gras d'un carboxylate d'ammonium quaternaire, de formule générale (I) suivante :

A⁻, R₁R₂R₃ N⁺ CH₂ C(O) O R₄ (I)

dans laquelle les radicaux R₁, R₂ et R₃, identiques ou différents, sont des radicaux choisis parmi l'hydrogène, les radicaux alkyles, linéaires ou ramifiés, en C₁ à C₁₂, substitués ou non substitués, et R₄ est une chaîne hydrocarbonée d'alcool gras en C₁₀ à C₂₂, linéaire ou ramifiée, substituée ou non substituée, saturée ou insaturée, A est un anion sulfonate, tel qu'un alkylsulfonate, de préférence un méthylsulfonate CH₃SO₃-.

Le carboxylate d'ammonium constitue une partie hydrophile de la molécule d'ester qui est soluble en solution aqueuse, alors que la chaîne hydrocarbonée d'alcool gras est hydrophobe. De préférence, l'ester d'alcool gras est un ester d'alcool gras d'une N,N,N trialkyl glycine, avec des radicaux alkyles en C₁ à C₃ identiques ou différents, de préférence un ester d'alcool gras de la N,N,N triméthyl glycine, encore appelé ester d'alcool gras de la glycine bétaïne. Contrairement à l'art antérieur, plus particulièrement le chapitre "Anti-mousses et agents démoussants" des Techniques de l'Ingénieur - J2 - 205 page 3, dans lequel Vance BERGERON indique que des alcools à courte chaîne hydrocarbonée (moins de 10 atomes de carbone) peuvent être ajoutés à des solutions aqueuses pour déstabiliser une mousse, et que par contre des alcools à chaîne plus longue ont un effet opposé et favorisent la stabilité des mousses, les inventeurs ont constaté, de manière surprenante, que les alcools gras, aptes à être libérés lors de l'hydrolyse de l'ester d'alcool gras en milieu basique, appartenant aux alcools en C₁₀ à C₂₂, de préférence en C₁₀ à C₁₈, primaires ou secondaires, saturés ou comportant au moins une double liaison sont particulièrement efficaces en tant qu'anti-mousse.

Les alcools gras préférés sont choisis parmi le décanol, l'undécanol, l'alcool laurique, le tétradécanol, l'alcool oléique, le 10-undécène-1-ol, et le 2-décanol. Un avantage complémentaire est que ces alcools gras libérés sont des molécules naturelles et/ou peu toxiques pour l'environnement, de même que la N, N, N trialkyl glycine, extraite de la betterave. Le procédé de mise en oeuvre de l'additif anti-mousse, selon l'invention, consiste à introduire l'ester d'alcool gras seul dans la composition cimentaire sous forme d'une solution aqueuse de superplastifiant de pH acide, de préférence de pH inférieur à 6. Il n'est pas exclu d'introduire cet additif également sous forme de poudre dans la composition cimentaire, par exemple dans l'eau destinée au gâchage de la composition cimentaire.

La présente invention concerne également une solution aqueuse de superplastifiant pour composition cimentaire, caractérisée en ce que ladite solution aqueuse comprend au moins un superplastifiant, de préférence un superplastifiant choisi parmi des polycarboxylates avec des chaînes latérales polyoxyde d'éthylène, de préférence des poly(méth)acrylates avec des chaînes latérales polyoxyde d'éthylène, et au moins un additif anti-mousse sous forme d'ester d'alcool gras, l'alcool gras étant une molécule active anti-mousse.

Ainsi une solution homogène renfermant à la fois un superplastifiant et un additif anti-mousse peut être commercialisée et mise en oeuvre aisément soit dès le gâchage (introduite par exemple avec l'eau de gâchage) soit lors du malaxage de la composition cimentaire, se présentant sous forme de coulis, mortier ou béton.

De manière avantageuse, l'additif anti-mousse est présent dans la solution aqueuse superplastifiante en une concentration comprise entre 0,01 % et 5 % en poids, de préférence comprise entre 0,1 et 2 % en poids, de préférence encore comprise entre 0,1 % et 0,6 % en poids, par rapport au superplastifiant.

Enfin, l'additif anti-mousse décrit ci-dessus seul, sous forme de poudre ou de solution aqueuse, avantageusement en solution dans une solution aqueuse de superplastifiant telle que décrite ci-dessus trouve une utilisation intéressante dans les mortiers ou les bétons pour réduire la quantité d'air entraînée lors du gâchage et du malaxage de la composition cimentaire, et plus particulièrement pour limiter la quantité d'air occlus dans un mortier frais à une valeur inférieure à 6 % volumique, de préférence inférieure à 4,5 % volumique (mesurées selon la norme NF EN 1015-7 méthode A) ou dans un béton frais à une valeur inférieure à 3 % volumique.

L'utilisation de ce précurseur anti-mousse est ainsi adaptée à de nombreux cas où la composition cimentaire renferme des adjuvants pouvant entraîner de l'air lors de son malaxage.

La présente invention concerne également un procédé de limitation de l'inclusion d'air dans un mortier frais à une valeur inférieure à 6 % volumique, de préférence inférieure à 4,5 % volumique (valeur mesurée selon la norme NF EN 1015-7 méthode A) ou dans un béton frais à une valeur inférieure à 3 % volumique, caractérisé en ce qu'il comprend l'incorporation à la composition cimentaire du mortier ou du béton lors du malaxage, dans l'eau de gâchage ou après l'ajout de l'eau de gâchage, d'une solution aqueuse d'un ester d'alcool gras, telle que décrite ci-dessus, de préférence sous la forme d'une solution aqueuse à pH<6renfermant un superplastifiant et l'additif anti-mousse sous forme d'ester d'alcool gras.

La concentration en superplastifiant pouvant varier de 0,05 à 5 %, de préférence de 0,1 à 2 % dans la composition cimentaire, la teneur en ester d'alcool gras selon la présente invention est avantageusement comprise entre 0,01 % et 5 % en poids, de préférence comprise entre 0,1 et 2 % en poids, de préférence encore comprise entre 0,1 % et 0,6 % en poids, par rapport au superplastifiant.

La présente invention va être illustrée par des exemples non limitatifs décrits ci-après, en référence à la figure 1 qui représente un additif anti-mousse selon la présente invention.

Les matériaux et molécules mis en oeuvre dans les exemples selon l'invention et les exemples comparatifs, ainsi que les méthodes d'évaluation de leurs performances sont les suivantes :

### 1- Matériaux mis en oeuvre

### •Solution de superplastifiant

Le superplastifiant utilisé est un polymère anionique de type polycarboxylate (PC) substitué par des chaînons de polyoxyde d'éthylène (POE) dont la formule chimique (II) est la suivante :

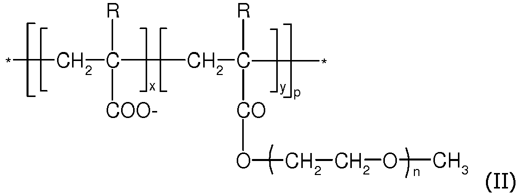

Dans les exemples ci-après R est CH₃, il s'agit donc d'un polyméthacrylate, avec un taux de greffage de POE (y) de 28%, n la longueur de chaîne des POE étant de 15, x = 72 % puisque x + y = 100 et p étant compris entre 10 et 100, et ici voisin de 50. Ce polymère est mis en solution dans l'eau selon une concentration égale à 30 % en poids. Le pH de cette solution est compris entre 5 et 6.

### •Additif anti-mousse

Un schéma illustrant une molécule d'additif anti-mousse conforme à la présente invention est représentée sur la figure 1.

L'additif anti-mousse est une molécule comportant à la fois une tête polaire 1 et une chaîne hydrophobe 3, les deux étant liés par une fonction carboxylate dont une des liaisons est, selon la flèche 2, hydrolysable à pH basique, c'est-à-dire au pH des formulations de ciment voisines de 12,5, permettant de libérer la chaîne hydrophobe.

Un schéma illustrant cette molécule est représentée sur la figure 1.

Dans les exemples présentés, la tête polaire et la glycine bétaïne de formule (III) ci-dessous qui est un triméthylalkylammoniumquaternaire avec une fonction carboxylate.

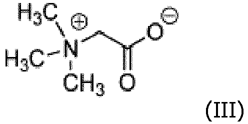

Du fait de la proximité des charges positive sur l'azote et négative de la fonction carboxylate, la glycine bétaïne est très stable en milieu acide, notamment au pH de la solution de superplastifiants. Pour renforcer la stabilité et garantir une stabilité sur une longue période, il est possible d'acidifier encore la solution de superplastifiant.

Sur la fonction carboxylate différentes molécules portant des chaînes hydrophobes, telles que différents alcools gras à différentes longueurs de chaînes ont été greffés. Ces esters ont été préparés selon la méthode décrite dans le brevet EP 1.742.999 B1. Ces esters de la glycine bétaïne sont stables à pH acide et sont hydrolysés à des pH supérieurs à 6,5, libérant l'alcool correspondant (voir, par exemple, l'étude de stabilité du mésylate de bétaïnate d'octadécyle dans ce brevet).

Au pH des compositions cimentaires (pH > 12), l'hydrolyse est quasi complète et instantanée. On libère alors l'alcool gras et la glycine bétaïne.

La réaction IV ci-après illustre l'hydrolyse d'un ester de glycine bétaïne avec l'alcool oléique :

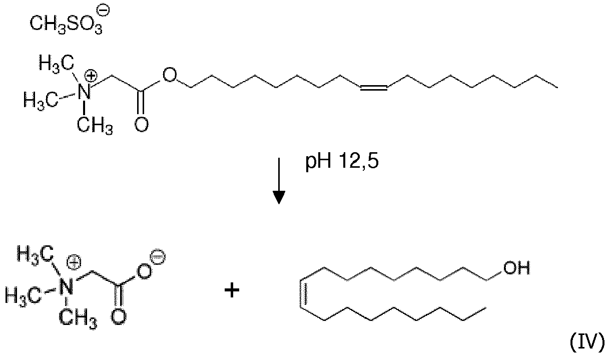

Les alcools gras testés sont à la fois des alcools primaires saturés ou insaturés et un alcool secondaire.

L'additif selon l'invention, sous la forme d'ester précurseur de la molécule active anti-mousse a été dissous dans la solution aqueuse de superplastifiant. Les pourcentages d'ester sont exprimés en pourcentage en poids par rapport au poids du superplastifiant dans ladite solution aqueuse.

Une fraction de cette solution aqueuse renfermant le superplastifiant et le précurseur d'anti-mousse a été ajoutée à une composition cimentaire sous la forme d'un mortier de béton équivalent (MBE : voir paragraphe suivant) lors du malaxage.

### •Mortier de béton équivalent (MBE)

La composition cimentaire sur laquelle ont été testées les différentes solutions aqueuses de mélange de superplastifiant avec différentes quantités d'additif ont été testées sur un mortier de béton équivalent (MBE* mis au point par le CTG). Cette composition de mortier est la suivante :
- 680g de ciment (CEM I 52,5)
- 1350 g de sable MBE*
- 303 g d'eau,
- 2 g de superplastifiant PC-POE, soit 0,3 % de la masse de ciment
(* La méthode du mortier de béton équivalent (MBE). Un nouvel outil d'aide à la formulation des bétons adjuvantés. A. Schwatzentruber, C. Catherine, Materials and structures, Vol. 33, October 2000, pp475-482)

Le mode opératoire détaillé de préparation du mortier de béton équivalent est décrit dans le document ci-dessus, le superplastifiant étant ici ajouté après l'eau de gâchage (c'est-à-dire "en différé") en fin de malaxage.

### 2- Méthodes d'évaluation

- La mesure d'étalement du mortier frais a été réalisée selon la méthode décrite dans l'article ci-dessus (Méthode du mortier de béton équivalent).
   Tous les essais réalisés ont été mis en oeuvre de manière à ce que l'étalement de référence en millimètres soit toujours voisin de 210 mm avec le superplastifiant sans ajout de l'additif anti-mousse.
   En conséquence, la concentration en superplastifiant par rapport au ciment est toujours restée identique (0,3 % en poids par rapport au ciment) dans l'ensemble des essais (exemples comparatifs - exemples selon l'invention) présentés ci-après.
- Pour l'évaluation de l'efficacité de l'anti-mousse, la norme NF EN 1015-7 méthode A a été mise en oeuvre. Les mesures de quantité d'air occlus dans le mortier ont été réalisées sur le mortier frais (MBE*) à la fin du malaxage.

### Exemples comparatifs :

Dans ces exemples ont été testées séparément les molécules de glycine bétaïne et d'alcool oléique (alcool gras en C₁₈), seules ou en combinaison, solubilisées dans une solution aqueuse à 30 % en poids du superplastifiant décrit au point 1- ci-dessus. Les résultats des tests effectués sur le mortier MBE sont regroupés dans le Tableau 1.

**Tableau 1**

| **Formulation** | **0** | **1** | **2** | **3** | **4** | **5** | **6** |
|---|---|---|---|---|---|---|---|
| % alcool oléique | / | 0,20 | / | 0,20 | 0,40 | / | 0,40 |
| % glycine bétaïne | / | / | 0,09 | 0,09 | / | 0,175 | 0,175 |
| **Résultats** | | | | | | | |
| Etalement (mm) | 199 | 222 | 218 | 223 | 229 | 213 | 235 |
| Air occlus (% volume) | 10 | 6,2 | 9,8 | 4,2 | 2,6 | 12 | 2,4 |

- Les pourcentages d'alcool oléique et de glycine bétaïne sont exprimés en poids par rapport au superplastifiant)

La précision de la mesure d'étalement est de plus ou moins 10 mm, pour l'air occlus cette précision est de plus ou moins 2 % (pour les valeurs d'air supérieures à 7 %) et l'incertitude devient plus ou moins 0,5 % pour les valeurs faibles).

D'après ces essais, on constate que l'ajout de l'alcool oléique diminue significativement l'air entraîné, la valeur passant de 10 % à environ 6 % à 0,2 % d'alcool oléique et tombant à une valeur proche de 2 % avec 0,4 % d'alcool oléique.

L'effet de la glycine bétaïne seule est négligeable, mais serait plutôt positive en synergie avec l'alcool oléique.

### Exemples selon l'inyention :

Dans ces exemples l'alcool gras n'a pas été ajouté directement dans la solution de superplastifiant mais solubilisé sous la forme de son précurseur, l'ester de glycine bétaïne d'alcool gras.

Les différents esters testés ainsi que les valeurs observées pour différentes quantités d'esters par rapport au superplastifiant sont regroupés dans le Tableau 2.

La teneur en superplastifiant était de 0,3 % poids/ciment. La quantité d'ester est exprimée en % pondéral par rapport au superplastifiant (valeur de l'extrait sec de la solution aqueuse).

**Tableau 2**

| **Composés (selon l'invention)** | **Etalement (mm)** | **Air entraîné (%)** |
|---|---|---|
| Superplastifiant seul | 214 | 12 |
| + ester de la glycine bétaïne (oléique) C₁₈ insaturé | | |
| 0,10 % | 208 | 11,5 |
| 0,20 % | 209 | 12,5 |
| 0,41 % | 212 | 12 |
| 1,14 % | 206 | 11,5 |
| 2,09 % | 211 | 8,5 |
| + ester de la glycine bétaïne (Laurique) C₁₂ | | |
| 0,59 % | 218 | 5,6 |
| 2,00 % | 220 | 3,2 |
| + ester de la glycine bétaïne (Décanol) C₁₀ | | |
| 0,62 % | 225 | 7,2 |
| 2% | 214 | 3,2 |
| + ester de la glycine bétaïne (tetradécanol) C₁₄ | | |
| 0,40 % | 206 | 7,6 |
| 2,00 % | 218 | 4,2 |
| + ester de la glycine bétaïne (undécanol) C₁₁ | | |
| 0,44 % | 211 | 6,4 |
| 2,00 % | 209 | 3,2 |
| + ester de la glycine bétaïne (10-undecan-1-ol) C₁₁ insaturé | | |
| 0,43 % | 201 | 12,5 |
| 2,00 % | 217 | 2,8 |
| + ester de la glycine bétaïne (2-décanol) C₁₀ secondaire | | |
| 0,38 | 211 | 13 |
| 2,00 | 221 | 3 |
| **Exemple comparatif** | | |
| Superplastifiant | | |
| + dérivé amide de la glycine bétaïne (oléique) | | |
| 1 % | 202 | 15 |

Dans ce tableau 2, on remarque que l'étalement n'est pratiquement pas modifié quelle que soit la nature de l'ester et quelle que soit sa concentration. Cependant, les valeurs d'air entraîné sont variables mais dans la plupart des cas une valeur inférieure à 10 % est observée.

Les valeurs les plus faibles inférieures à 4,5 % d'air occlus sont obtenues pour des concentrations voisines de 2 % en poids de l'ester par rapport au superplastifiant et pour des esters préparés avec des alcools gras en C₁₀ à C₁₄.

A titre de comparaison, une amide pouvant libérer une amine en C18 (oléique) a été testée à 1 % en poids par rapport au superplastifiant dans une solution de superplastifiant. La quantité d'air entraîné mesurée était de 15 % soit une valeur très supérieure à la valeur de 12 % de référence avec le superplastifiant seul. L'amide a donc un effet inverse (c'est-à-dire une augmentation de l'air entraîné) de celui de l'ester d'alcool gras de la présente invention.

De plus, les amides sont stables jusqu'à un pH basique, ne libérant les amines grasses qu'au-delà d'un pH d'environ 8 ou 9, alors que les alcools gras sont libérés à partir des esters selon l'invention à partir d'un pH supérieur ou égal à 6,5, l'hydrolyse du précurseur étant totale à pH 9. L'agent actif selon la présente invention est donc disponible plus rapidement dans la composition cimentaire, avant même la formation des bulles (mousse).

On voit donc que ces esters d'alcool gras qui présentent une grande solubilité dans la solution aqueuse de superplastifiant sont tout à fait appropriés pour limiter, voire diminuer, la teneur d'air entraîné dans des compositions cimentaires.

Il a été observé que les solutions aqueuses de superplastifiant et du précurseur d'anti-mousse selon l'invention sont parfaitement stables à des pH ≤ 5,5 et ne présentent aucun trouble ni séparation de phase, pendant au moins un an.

## Revendications

1. Composition cimentaire, à base de ciment et d'eau, préparée avec adjonction de superplastifiant, **caractérisée en ce qu'**elle renferme un additif anti-mousse se présentant sous la forme d'un ester d'alcool gras soluble en milieu aqueux à pH acide et hydrolysable lors de son incorporation dans ladite composition cimentaire, apte à libérer ledit alcool gras, l'alcool gras étant une molécule active anti-mousse.

2. Composition cimentaire selon la revendication 1, **caractérisée en ce que** l'ester d'alcool gras est un ester d'alcool gras d'un carboxylate d'ammonium quaternaire, de formule générale (I) suivante :
A⁻, R₁R₂R₃ N⁺ CH₂ C(O) O R₄ (I)
dans laquelle les radicaux R₁, R₂ et R₃, identiques ou différents, sont des radicaux choisis parmi l'hydrogène, les radicaux alkyles, linéaires ou ramifiés, en C₁ à C₁₂, substitués ou non substitués, et R₄ est une chaîne hydrocarbonée d'alcool gras en C₁₀ à C₂₂, linéaire ou ramifiée, substituée ou non substituée, saturée ou insaturée, A est un anion sulfonate, tel qu'un alkylsulfonate, de préférence un méthylsulfonate.

3. Composition cimentaire selon la revendication 2, **caractérisée en ce que** l'ester d'alcool gras est un ester d'alcool gras d'une N,N,N trialkyl glycine, avec des radicaux alkyles en C₁ à C₃ identiques ou différents, de préférence un ester d'alcool gras de la N,N,N triméthyl glycine.

4. Composition cimentaire selon l'une quelconque des revendications précédentes, **caractérisée en ce que** l'alcool gras (apte à être libéré lors de l'hydrolyse de l'ester en milieu basique) est un alcool en C₁₀ à C₂₂, de préférence en C₁₀ à C₁₈, primaire ou secondaire, saturé ou comportant au moins une double liaison.

5. Composition cimentaire selon l'une quelconque des revendications précédentes, **caractérisée en ce que** l'alcool gras est choisi parmi le décanol, l'undécanol, l'alcool laurique, le tétradécanol, l'alcool oléique, le 10-undécène-1-ol, et le 2-décanol.

6. Solution aqueuse de superplastifiant pour composition cimentaire, **caractérisée en ce que** ladite solution aqueuse comprend, solubilisés dans ladite solution aqueuse, au moins un superplastifiant, de préférence un superplastifiant choisi parmi des polycarboxylates avec des chaînes latérales polyoxyde d'éthylène, de préférence des poly(méth)acrylates avec des chaînes latérales polyoxyde d'éthylène, et au moins un additif anti-mousse sous forme d'un ester d'alcool gras soluble en milieu aqueux à pH acide et hydrolysable lors de son incorporation dans ladite composition cimentaire, apte à libérer ledit alcool gras, l'alcool gras étant une molécule active anti-mousse.

7. Solution aqueuse selon la revendication 6, **caractérisée en ce que** la concentration en additif anti-mousse est comprise entre 0,01 % et 5 % en poids, de préférence comprise entre 0,1 et 2 % en poids, de préférence encore comprise entre 0,1 % et 0,6 % en poids, par rapport au superplastifiant.

8. Utilisation de la solution selon l'une quelconque des revendications 6 à 7 dans des mortiers ou des bétons, pour réduire la quantité d'air entraînée lors du gâchage et du malaxage de la composition cimentaire.

9. Utilisation selon la revendication 8 pour limiter la quantité d'air occlus dans un mortier frais à une valeur inférieure à 6 % volumique, de préférence inférieure à 4,5 % volumique (valeur mesurée selon la norme NF EN 1015-7 méthode A) ou dans un béton frais à une valeur inférieure à 3 % volumique.

10. Procédé de limitation de l'inclusion d'air dans un mortier frais à une valeur inférieure à 6 % volumique, de préférence inférieure à 4,5 % volumique (valeur mesurée selon la norme NF EN 1015-7 méthode A) ou dans un béton frais à une valeur inférieure à 3 % volumique, **caractérisé en ce qu'**il comprend l'incorporation à la composition cimentaire du mortier ou du béton lors du malaxage, dans l'eau de gâchage ou après l'ajout de l'eau de gâchage, d'une solution aqueuse selon l'une quelconque des revendications 6 ou 7 renfermant un superplastifiant et l'additif anti-mousse sous forme d'ester d'alcool gras.

## Patentansprüche

1. Zementzusammensetzung auf der Basis von Zement und von Wasser, hergestellt unter Hinzufügung von Superverflüssiger, **dadurch gekennzeichnet, dass** sie einen Schaumhemmungszusatz in Form eines Fettalkoholesters umfasst, in wässrigem Milieu bei saurem pH löslich und wasserlöslich bei seiner Einarbeitung in die Zementzusammensetzung, imstande, den Fettalkohol freizusetzen, wobei der Fettalkohol ein aktives Schaumhemmungsmolekül ist.

2. Zementzusammensetzung nach Anspruch 1, **dadurch gekennzeichnet, dass** der Fettalkoholester ein Fettalkoholester eines quarternären Ammoniumcarboxylats mit der folgenden allgemeinen Formel (I)
A⁻, R₁R₂R₃ N⁺ CH₂ C(O) O R₄ (I)
ist, wobei die Radikale R₁, R₂ und R₃, identisch oder unterschiedlich, Radikale sind, die aus dem Wasserstoff, den linearen oder verzweigten, substituierten oder nicht substituierten C₁-C₁₂-Alkylradikalen ausgewählt sind, und R₄ eine lineare oder verzweigte, substituierte oder nicht substituierte, gesättigte oder ungesättigte C₁₀-C₂₂-Kohlenwasserstoff-Fettalkoholkette ist, wobei A ein Sulfonatanion wie ein Alkylsulfonat, vorzugsweise ein Methylsulfonat, ist.

3. Zementzusammensetzung nach Anspruch 2, **dadurch gekennzeichnet, dass** der Fettalkoholester ein Fettalkoholester eines N,N,N Trialkylglycins mit identischen oder unterschiedlichen C₁-C₃-Alkylradikalen, vorzugsweise ein Fettalkoholester des N,N,N Trimethylglycins, ist.

4. Zementzusammensetzung nach einem der vorangehenden Ansprüche, **dadurch gekennzeichnet, dass** der Fettalkohol (der nach der Hydrolyse des Esters in basischem Milieu freisetzbar ist) ein primärer oder sekundärer, gesättigter oder aufweisend mindestens eine Doppelbindung, C₁₀-C₂₂-, vorzugsweise C₁₀-C₁₈-Alkohol ist.

5. Zementzusammensetzung nach einem der vorangehenden Ansprüche, **dadurch gekennzeichnet, dass** der Fettalkohol aus dem Decanol, dem Undecanol, dem Laurinalkohol, dem Tetradecanol, dem Oleinalkohol, dem 10-Undecen-1-ol und dem 2-Decanol ausgewählt ist.

6. Wässrige Superverflüssigerlösung für Zementzusammensetzung, **dadurch gekennzeichnet, dass** die wässrige Lösung umfasst, gelöst in der wässrigen Lösung, mindestens einen Superverflüssiger, vorzugsweise einen Superverflüssiger, ausgewählt aus den Polycarboxylaten mit Ethylenpolyoxid-Seitenketten, vorzugsweise den Poly(meth)acrylaten mit Ethylenpolyoxid-Seitenketten, und mindestens einen Schaumhemmungszusatz in Form eines Fettalkoholesters, in wässrigem Milieu bei saurem pH löslich und bei seiner Einarbeitung in die Zementzusammensetzung wasserlöslich, imstande, den Fettalkohol freizusetzen, wobei der Fettalkohol ein aktives Schaumhemmungsmolekül ist.

7. Wässrige Lösung nach Anspruch 6, **dadurch gekennzeichnet, dass** die Schaumhemmungszusatzkonzentration zwischen 0,01 und 5 Gew.-% inklusive, vorzugsweise zwischen 0,1 und 2 Gew.-% inklusive, noch vorzugsweiser zwischen 0,1 und 0,6 Gew.-% inklusive in Bezug zum Superverflüssiger beträgt.

8. Verwendung der Lösung nach einem der Ansprüche 6 bis 7 in Mörteln oder in Beton zur Reduzierung der Menge der Luft, die beim Anmachen und Mischen der Zementzusammensetzung mitgenommen wird.

9. Verwendung nach Anspruch 8 zwecks Begrenzung der in einem frischen Mörtel eingeschlossenen Luftmenge auf einen Wert unter 6 Vol.-%, vorzugsweise unter 4,5 Vol.-% (nach Norm NF EN 1015-7 Methode A gemessener Wert) oder in einem frischen Beton auf einen Wert unter 3 Vol.-%.

10. Verfahren zur Begrenzung von Lufteinschluss in einem frischen Mörtel auf einen Wert unter 6 Vol.-%, vorzugsweise unter 4,5 Vol.-% (nach Norm NF EN 1015-7 Methode A gemessener Wert) oder in einem frischen Beton auf einen Wert unter 3 Vol.-%, **dadurch gekennzeichnet, dass** es die Einarbeitung in die Zementzusammensetzung des Mörtels oder des Betons beim Mischen, in das Anmachwasser oder nach dem Hinzufügen des Anmachwassers einer wässrigen Lösung nach einem der Ansprüche 6 oder 7 umfasst, die einen Superverflüssiger und den Schaumhemmungszusatz in Form von Fettalkoholester umfasst.

## Claims

1. Cement- and water-based cement composition prepared with the addition of a superplasticizer, **characterized in that** it contains a defoaming additive in the form of a fatty alcohol ester soluble in an aqueous medium at acid pH and hydrolysable when incorporated in said cement composition, able to release said fatty alcohol, the fatty alcohol being an active defoaming molecule.

2. The cement composition according to claim 1, **characterized in that** the fatty alcohol ester is a fatty alcohol ester of a quaternary ammonium carboxylate of following general formula (I)
A-, R₁R₂R₃ N⁺ CH₂ C(O) O R₄ (I)
where the radicals R₁, R₂ and R₃, the same or different, are radicals selected from among hydrogen, C₁ to C₁₂ alkyl radicals, straight-chain or branched, substituted or unsubstituted, and R₄ is a C₁₀ to C₂₂ fatty alcohol hydrocarbon chain, straight-chain or branched, substituted or unsubstituted, saturated or unsaturated, A is a sulfonate anion such as an alkylsulfonate, preferably a methylsulfonate.

3. The cement composition according to claim 2, **characterized in that** the fatty alcohol ester is a fatty alcohol ester of an N,N,N trialkyl glycine, with C₁ to C₃ alkyl radicals, the same or different, preferably a fatty alcohol ester of N,N,N trimethyl glycine.

4. The cement composition according to any of the preceding claims, **characterized in that** the fatty alcohol (able to be released during hydrolysis of the ester in a basic medium) is a C₁₀ to C₂₂ preferably C₁₀ to C₁₈ primary or secondary alcohol, saturated or comprising at least one double bond.

5. The cement composition according to any of the preceding claims, **characterized in that** the fatty alcohol is selected from among decanol, undecanol, lauric alcohol, tetradecanol, oleic alcohol, 10-undecene-1-ol and 2-decanol.

6. Aqueous superplasticizer solution for cement composition, **characterized in that** said aqueous solution contains at least one superplasticizer solubilised in said aqueous solution, preferably a superplasticizer selected from among polycarboxylates with poly(ethylene oxide) side chains, preferably poly(meth)acrylates with poly(ethylene oxide) side chains, and at least one defoaming additive in the form of a fatty alcohol ester soluble in an aqueous medium at acid pH and hydrolysable when incorporated in said cement composition, able to release said fatty alcohol, the fatty alcohol being an active defoaming molecule.

7. The aqueous solution according to claim 6, **characterized in that** the concentration of defoaming additive is between 0.01 % and 5 % by weight, preferably between 0.1 and 2 % by weight, further preferably between 0.1 % and 0.6 % by weight relative to the superplasticizer.

8. Use of the solution according to any of claims 6 to 7 in mortars or concretes to reduce the quantity of entrained air when adding water and mixing the cement composition.

9. The use according to claim 8, to limit the amount of occluded air in fresh mortar to a value below 6 % by volume, preferably lower than 4.5 % by volume (value measured as per standard NF EN 1015-7, method A) or in fresh concrete to a value below 3 % by volume.

10. Method to limit the inclusion of air in fresh mortar to a value below 6 % by volume, preferably lower than 4.5 % by volume (value measured as per standard NF EN 1015-7, method A) or in fresh concrete to a value below 3 % by volume, **characterized in that** it comprises the incorporation in the cement composition of the mortar or concrete, in the mixing water when mixing or after adding the mixing water, of an aqueous solution according to any of claims 6 or 7 containing a superplasticizer and the defoaming additive in the form of a fatty alcohol ester.
